# EUROPÄISCHE PATENTSCHRIFT

(11) **EP 2 129 645 B1**
(45) Veröffentlichungstag und Bekanntmachung des Hinweises auf die Patenterteilung: **07.05.2014**
(21) Anmeldenummer: 08718092.3
(22) Anmeldetag: 20.03.2008
(51) Int. Cl.: C07C 29/76, C07C 31/22, B01J 21/18, B01J 35/10

(54) **VERFAHREN ZUR FARBZAHLVERBESSERUNG VON TRIMETHYLOLPROPAN**
METHOD FOR IMPROVING THE COLOR NUMBER OF TRIMETHYLOLPROPANE
PROCÉDÉ D'AMÉLIORATION DE L'INDICE DE COULEUR DU TRIMETHYLOLPROPANE

(30) Priorität: 23.03.2007 DE 102007013963
(43) Veröffentlichungstag der Anmeldung: 09.12.2009
(73) Patentinhaber: LANXESS Deutschland GmbH, 50569 Köln (DE)
(72) Erfinder: MÄGERLEIN, Wolfgang, 68165 Mannheim (DE); NOTHEIS, Ulrich, 41539 Dormagen (DE); FRIEDERICH, Michael, 47800 Krefeld (DE); GERRIETS, Hans-Dieter, 47239 Duisburg (DE); GRIZINIA, Heinrich, 41812 Erkelenz (DE); WAGNER, Paul, 40597 Düsseldorf (DE)
(86) Internationale Anmeldenummer: PCT/EP2008/053376
(87) Internationale Veröffentlichungsnummer: WO 2008/116826

(56) Entgegenhaltungen:
- DE-A1- 10 029 055
- US-A- 3 037 060
- US-A1- 2004 225 162

## Beschreibung

Die vorliegende Erfindung betrifft ein Verfahren zur Farbzahlverbesserung von Trimethylolpropan mit einem Trimethylolpropan- Gehalt von 90 - 100 Gew.% mit geringer Farbzahl durch Aktivkohlebebandlung.

Trimethylolpropan, im folgenden TMP genannt, findet breite industrielle Verwendung zur Herstellung von Polyestern, Polyurethanen, Polyethern, Schaumkunststoffen, Weichmachern, Alkydharzen, Schutzanstrichen, Schmiermitteln, Appreturen und Elastomeren. Ferner substituiert es Glycerin in einigen industriellen Anwendungen.

In technischem Maßstab wird TMP überwiegend durch zwei alternative Verfahren hergestellt.

Das sogenannte anorganische Cannizzaro-Verfahren umfasst zunächst im ersten Schritt eine Aldolkondensation von Butanal und Formaldehyd und in einem zweiten Schritt eine gekreuzte Cannizzaro-Reaktion zwischen dem Aldolkondensationsprodukt des ersten Schrittes und Formaldehyd in Gegenwart von stöchiometrischen Mengen einer anorganischen Base wie beispielsweise Natrium- oder Calciumhydroxid. Das im ersten Schritt als Aldolkendensationsprodukt gebildete Dimethylolbutanal reagiert Dabei in der zweiten Stufe mit dem überschüssigen Formaldehyd in einer Dispropörtionierungsreaktion zu TMP und je nach eingesetzter Base, dem entsprechenden Formiat, wie zum Beispiel Natrium- oder Calciumformiat. In einer Abwandlung, dem sogenannten organischen Cannizzaro-Verfahren, wird im zweiten Schritt anstelle einer anorganischen Base ein tertiäres Alkylamin verwendet. Damit lassen sich üblicherweise höhere Ausbeuten erzielen als mit einer anorganischen Base. Es fällt als Nebenprodukt Trialkylammoniumformiat an.

Weiterhin ist die Herstellung von TMP durch das so genannte Hydrierverfahren möglich. Dabei wird Formaldehyd mit Butanal in Gegenwart katalytischer Mengen eines Amins zu Dimethylolbutanal umgesetzt. Nach Abtrennung überschüssigen Formaldehyds wird das Reaktionsgemisch einer Hydrierung unterworfen, bei der das gewünschte TMP erhalten wird.

Kommerziell erhältliche TMP-Quälitäten weisen üblicherweise eine Färbung auf, die durch das Vorliegen von Verunreinigungen verursacht wird. Bei einigen Anwendungen wie zum Beispiel die Herstellung von besonders transparenten Polyestern oder bestimmten Lackrohstoffen ist diese Färbung jedoch störend. In der Literatur sind verschiedene Verfahren beschrieben, mit denen eine Farbzahlverbesserung von TMP erreicht werden soll.

US 3,097,245 beschreibt ein Verfahren zur Herstellung von TMP mit einer APHA-Farbzahl zwischen 50 und 200. Diese Farbzahl wird dabei durch das Einhalten bestimmter Reaktionsbedingungen der Cannizarro-Reaktion hinsichtlich Temperatur, Reaktionszeit, pH-Wert und Konzentration der Ausgangsverbindungen erreicht. Weiterhin schließt sich an der Reaktion die Behandlung der erhaltenen Lösung mit einem Ionenaustauscherharz an.

US 5,603,835 offenbart ein Verfahren zur Herstellung von TMP mit Alpha-farbzahlen von weniger als 100 durch extraktive Nachbehandlung von rohen TMP-Lösungen mit einem Ether oder einem Ester.

Die beiden vorstehend beschriebenen Verfahren zur TMP-Farbzahlverbesserung weisen den Nachteil auf, dass sie technisch aüfwändig sind, da bestimmte Bedingungen genau eingehalten werden müssen und zusätzlich den Einsatz eines Ionenaustauscherharzes bzw. die Einführung mindestens eines Lösungsmittels erfordern.

SU-A 125552 beschreibt eine Hydrierung zur Reinigung von TMP, das nach dem Cannizzaro-Verfahren hergestellt wurde. Man erhält durch Hydrierung an Nickel-, Zink-, Molybdän- oder Kupferkatalysatoren TMP mit einem Gehalt von ca. 98 % nach Destillation. Es wird erwähnt, dass das erhaltene TMP farblos ist, wobei jedoch keine Farbzahl angegeben wird. Es hat sich jedoch in der Praxis gezeigt, dass die mit diesem Verfahren erhältliche Farbzahl für viele Zwecke nicht ausreichend ist.

Ein weiteres Verfahren zur Farbzahlverbesserung von TMP durch Hydrierung ist in der Anmeldung DE 199 63 442 beschrieben. TMP wird hierbei nach der Herstellung destillativ gereinigt und anschließend vorzugsweise mit einem heterogenen Katalysator unter Wasserstoffdruck behandelt. Die durch Hydrierung erreichte Farbzahl kann durch vorheriges mehrmaliges Destillieren weiter verbessert werden.

Nachteilig bei allen Hydrierverfahren zur Farbzahlverbesserung ist jedoch der hohe apparative Aufwand sowie eine Qualitätsminderung des erhaltenen TMP durch Katalysatorabrieb. Ferner vermindern die Kosten für den Katalysator die wirtschaftliche Effizienz.

In der DE 100 29 055 ist ein Verfähren zur Farbzahlverbesserung von TMP beschrieben, bei dem destillativ vorgereinigtes TMP mit einer Reinheit von vorzugsweise > 95 % einer Temperung, vorzugsweise bei Temperaturen von 160 bis 240 °C unterworfen wird und das TMP anschließend erneut, vorzugsweise durch Destillation, gereinigt wird. Durch den Temperschritt werden die farbzahlgebenden Nebenkomponenten in höher siedende, schwerflüchtige Komponenten überführt. Nachteilig bei diesem Verfahren ist jedoch, dass sich an die Temperung erneut ein Reinigungsschritt anschließen muss, beispielsweise eine Destillation, bzw. dieser Reinigungsschritt mit der Temperung gekoppelt werden muss, um diese höher siedenden Nebenkomponenten zu entfernen und ein TMP mit niedriger Farbzahl zu erhalten.

Ein weiteres Verfahren zur Farbzahlverbesserung von Polyalkoholen verwendet Aktivkohle zur Entfernung der Verunreinigungen. Ein entsprechendes Verfahren für Pentaerythrit ist beispielweise in H. Mouren et al., Mem. Poudres 32, 89 (1950) sowie in Org. Synth., Collect. Vol. 1, 425 beschrieben.

In der CZ 184381 werden zur Reinigung von TMP und anderen Polyalkoholen oxidation-und/oder Reduktionsmittel wie z.B. Kaliumpermanganat und Natriwnsulfit verwendet, vorzugsweise in Gegenwart von Aktivkohle oder anderen Stoffen, die Verunreinigungen binden können. Es werden APHA-Farbzahlen von etwa > 100 erreicht.

Nachteilig bei den genannten Verfahren ist jedoch die Tatsache, dass sie in verdünnten Lösungen durchgeführt werden, was durch die Notwendigkeit der Abtrennung des Lösungsmittels vom Product unwirtschaftlich ist.

Es bestand demnach die Aufgabe, ein effizientes und ohne aufwändige Apparaturen durchführbares Verfahren zur Farbzahlverbesserung von TMP mit niedriger Farbzahl bereitzustellen, das die Nachteile des Standes der Technik überwindet.

Es wurde nun ein Verfahren zur Farbzahlverbesserung von TMP gefunden, das dadurch gekennzeichnet ist, dass bei Umgebungsdruck in einem Temperaturbereich von 80 - 200°C flüssiges TMP mit einem Trimethylolpropan- Gehalt von 90 - 100 Gew.% mit Aktivkohle in Kontakt gebracht wird und die Aktivkohle anschließend abgetrennt wird.

Vorzugsweise wird TMP verwendet das vor seiner Anwendung im erfindungsgemäßen Verfahren destilliert wurde.

Flüssig im Sinne der vorliegenden Erfindung bedeutet dass die Substanz, die mit der Aktivkohle in Kontakt gebracht wird weniger als 5 Gew.-%, vorzugsweise weniger als 1 Gew-% Feststoffe enthält. Feststoffe können beispielsweise nicht geschmolzenes TMP oder feste andere Stoffe sein.

Um den flüssigen Zustand zu erreichten, sind Bedingungen bevorzugt, in dem einerseits die dynamische Viskosität des TMP solche Werte aufweiset, die eine technische Verarbeitung wie Umpumpen, Rühren etc. im Wesentlichen problemlos gestatten. Bevorzugt ist dabei ein dynamischer Viskositätsbereich von 2.4 bis 160 cP, besonders bevorzugt ein dynamischer Viskositätsbereich von 11 bis 62 cP. Bei Umgebungsdruck entspricht dies einem Temperaturbereich von 80 bis 200°C, bevorzugt von 100 bis 150°C, besonders bevorzugt von 100 bis 120°C.

Das erfindungsgemäße Verfahren kann in diskontinuierlicher oder in kontinuierlicher Verfahrensweise durchgeführt werden.

Zur Durchführung in diskontinuierlicher Verfahrensweise kann man beispielsweise TMP aufschmelzen, die Schmelze mit einer bestimmte Menge Aktivkohle versetzen und nach einer bestimmten Kontaktzeit schließlich das flüssige TMP von der Aktivkohle wieder entfernten.

Vorzugsweise wird während des Inkontaktbringens eine ausreichende Durchmischung z.b. durch Rühren sichergestellt. Die Abtrennung der Aktivkohle kann z.B. durch Filtration, Zentrifugation oder Sedimentation erfolgen.

Zur Durchführung in kontinuierlicher Verfahrensweise kann flüssiges TMP beispielsweise durch eine Schüttung aus Aktivkohle in geradem Durchgang oder in Kreislauffahrweise gepumpt werden. Dabei ist beispielsweise eine Riesel- oder eine Sumpffahrweise möglich. In einer bevorzugten Form werden stückige Aktivkohlen als Festbett eingesetzt.

In einer Ausführungsform werden die Aktivkohlen von unten angeströmt und beim Anfahren der erste Teil des Produktstroms, der noch Aktivkohlepartikel enthält, zurückgeführt oder verworfen. Nach einiger Zeit kommt dann klares Produkt. Falls die Entfärbungskapazität der Aktivkohle erschöpft ist, kann die Aktivkohle regeneriert bzw. ausgetauscht werden. Die Regenerierung kann beispielweise durch thermische Behandlung oder durch Behandlung mit Dampf geschehen.

In einer weiteren Ausführungsform wird das flüssige TMP so über eine Reihe von hintereinandergeschalteten Aktivkohlebetten gefahren, dass ein oder mehrere Betten zur Reinigung genutzt werden, während gleichzeitig mindestens ein Aktivkohlebett regeneriert wird. Die Betten werden dabei der Reihe nach regeneriert. Die Regenerierung der Betten kann beispielweise erfolgen, indem man das flüssige TMP aus dem Bett ablaufen lässt und anschliessend die Aktivkohle mit Dampf behandelt.

Als Aktivkohlen können prinzipiell alle bekannten eingesetzt werden. Bevorzugte Aktivkohlen sind solche, die aus Holzkohle, Braunkohle, Steinkohle, Torf, Holz, Kokosnussschalen oder Olivenkernen hergestellt werden.

Die Aktivkohlen können durch Dampfaktivierung oder durch chemische Aktivierung hergestellt worden sein, wobei optional weitere Behandlungsschritte durchgeführt worden sein können, wie zum Beispiel Wäschen, pH-Anpassung, Imprägnierung, Temperung oder Oxidation bzw. Reduktion oder verschieden Verfahren zur Formgebung wie Granulieren, Extrudieren oder Pelletisieren.

Der Habitus der Aktivkohlen ist für den Einsatz im erfindungsgemäßen Verfahren prinzipiell nicht beschränkt. Vorzugsweise werden pulvrige, körnige, granulierte oder extrudierte Aktivkohlen eingesetzt.

In einer Ausführugsform der Erfindung werden Aktivkohlen verwendet die einen pH-Wert (wäßriger Auszug) im Bereich von 1 bis 12 bei Standardbedingungen aufweisen. Bevorzugt sind Aktivkohlen, die einen pH-Wert im Bereich von 4 bis 9 bei Standardbedingungen aufweisen,

Die verwendeten Aktivkohlen können beispielsweise weiterhin BET-Oberflächen im Bereich von 500 bis 2500 m²/g aufweisen. Bevorzugt sind Aktivkohlen, die BET-Oberflächen im Bereich von 700 bis 2000 m²/g aufweisen.

In einer bevorzugten Ausführungsform schließt sich das erfindungsgemäße Verfahren an einer Stelle im Herstellungsprozess an, in der das TMP geschmolzen vorliegt, wie zum Beispiel nach einer destillativen Reinigung.

Das Inkontaktbringen des im Wesentlichen reinen TMP mit Aktivkohle kann beispielsweise mehr als 30 s dauern, bevorzugt 1 min bis 24 h. Besonders bevorzugt sind Kontaktzeiten von 10 min bis 8 h, ganz besonders bevorzugt von 10 min bis 4 h.

Bei diskontinuierlicher Durchführung kann die, Menge an Aktivkohle bezogen auf eingesetztes TMP beispielsweise im Bereich von 0,01 Ges.-% bis 100 Gew.-% liegen. Bevorzugt ist eine Aküvkohlemenge von 0,1 Gew.-% bis 5 Gew.-%.

Bei kontinuierlicher Durchführung kann die spezifische Belastung beispielsweise im Bereich von 0,01 kg/(l*h) bis 10 kgl(l*h) liegen, bevorzugt ist ein Bereich von 0,1 kg((l*h) bis 3 kg/(l*h).

Das erfindungsgemäße Verfahren zur Farbzahlverbesserung von TMP mit Aktivkohle ist grundsätzlich für TMP jeglicher Herkunft anwendbar. Es kann TMP verwendet werden, das über das organische, das anorganische Cannizzaro-Verfahren oder das Hydrierverfahren hergestellt wurde. Vorzugsweise wird TMP eingesetzt, das durch ein anorganisches Cannizzaro-Verfahren hergestellt wurde. Typischerweise weist TMP, das über vorgenannte Methoden hergestellt wurde und gegebenenfalls weiteren Reinigungsschritten unterzogen wurde, zunächst APHA-Farbzahlen von 10 bis 1000 auf.

Das erfindungsgemäße Verfahren zeichnet sich dadurch aus, dass die APHA-Farbzahl von TMP auf effiziente Weise erheblich verbessert werden kann.

### Beispiele

### Allgemeines:

Die APHA-Farbzahlen [Einstufung der Farbe nach der Platin Cobalt Skala, vgl. DIN ISO 6271] wurde mit einem Photometer Lico 200 der Fa. Dr. Lange bestimmt. Dazu wurden jeweils 4 bis 5g TMP in eine 11 mm Rundküvette gefüllt, die Rundküvette mit einem Silikortstopfen verschlossen, bei 100°C aufgeschmolzen und vermessen. Für jede Probe wurden zwei Messungen durchgeführt und die Werte gemittelt.

### Beispiel 1:

Es wurden 150 g TMP mit einem Gehalt von 94.47 % (GC-Flächen-%) und einer APHA-Farbzahl von 243 aufgeschmolzen und auf eine Temperatur von 100°C gebracht. Anschließend wurden 0.75 g Aktivkohle zugesetzt und die Mischung 60 min bei dieser Temperatur gerührt. Anschließend wurde über eine beheizte Drucknutsche heiß abfiltriert und die APHA-Farbzahl bestimmt. Die Ergebnisse für verschiedene Aktivkohlen sind in der folgenden Tabelle wiedergegeben:

| Aktivkohle | APHA-Farbzahl nach Aktivkohlebehandlung |
|---|---|
| Dampfätiviertes, säuregewaschenes Aktivkohleextrudat mit BET-Oberfläche 1370 m²/g und 3 Gew-% Asche (Norit RX 3) | 144 |
| Dampfaktiviertes, säuregewaschenes Aktivkohlegranulat 0,4 bis 2 mm aus Kohle mit BET-Oberfläche 1125 m²/g, Jodzahl von 1025, Methylenblau-Absorption von 22 g/100g, und 0,5 Ges-% Asche. Die wässrige Aufschlämmung ist neutral (Norit GAC 1240+) | 97 |
| Dampfaktiviertes, säuregewaschenes Aktivkohlepulver 25 µ mit BET-Oberfläche 1000 m²/g, Jodzahl 250, Methylenblau-Absorption 18 g(100g, Melassezahl 900 [EUR] und 4 Gew-% Asche. Die wässrige Aufschlämmung ist neutra (Norit SX 1 G) | 22 |
| Dampfaktiviertes, säuregewaschenes Aktivkohlepulver 20 µ mit BET-Oberfläche 1100 m²/g, Jodzahl 1050, Methylenblau-Absorption 22 g/100g, Melassezahl 225 [EUR] und 5 Gew-°% Asche. Die wässrige Aufschlämmung ist neutral (Norit SX 1 G) | 23 |
| Dampfaktiviertes, säuregewaschenes Aktivkohleextrudat 0,8 mm mit BET-Oberfläche 1100 m²/g, Jodzahl 1000, Methylenblau-Absorption 22 g/100g, Melassezahl 350 [EUR] und 5 Gew-% Asche. Die wässrige Aufschlämmung ist neutral (Norit Rox 0.88) | 116 |
| Chemisch aktivierte, säuregewaschene, stückige Aktivkohle 3 mm mit Bentonit-Zusatz und BET-Oberfläche 1100 m²/g, Methylenblau-Absorption 25 g/100g, Melassezahl 350 [EUR] und 15 Gew-% Asche. Die wässrige Aufschlämmung ist sauer (pH 2 bis 3,5) (Norit Bentonorit CA 1) | 52 |

### Beispiel 2:

Es wurden 150 g TMP mit einem Gehalt von 99.23 % (GC-Fläehen-%) und einer ALPHA-Farbzahl von 10 aufgeschmolzen und auf eine Temperatur von 100°C gebracht. Anschließend wurden 0.75 g Aktivkohle zugesetzt und die Mischung 60 min bei dieser Temperatur geführt. Anschließend wurde über eine beheizte Drucknutsche heiß abfiltriert und die APHA-Farbzahl bestimmt. Die Ergebnisse für verschiedene Aktivkohlen sind in der folgenden Tabelle wiedergegeben:

| Aktivkohle | APHA-Farbzahl nach Aktivkohlebehandlung |
|---|---|
| Dampfaktiviertes, säure gewaschenes Aktivkohlegranulat (0,4 bis 2 mm) aus Kohle mit BET-Oberfläche 1125 m²/g, Jodzahl von 1025, Methylenblau-Absorption von 22 g/100g, und 0,5 Gew-% Asche. Die wässrige Aufschlemmung ist neutral (Norit GAC 1240+) | 0 |
| Dampfaktiviertes, säuregewaschenes Aktivkohlepulver (25 µ) mit BET-Oberfläche 1000 m²/g, Jodzahl 250, Methylenblau-Absorption 18 g/100g, Melassezahl 900 [EUR] und 4 Gew-% Asche. Die wässrige Aufschlämmung ist neutral (Norit SX 1 C) | 0 |
| Dampfaktiviertes, säuregewaschenes Aktivkohlepulver 20 µ mit BET-Oberfläche 1100 m²/g, Jodzahl 1050, Methyleblau-Absorption 22 g/100g, Melassezahl 225 [EUR] und 5 Gew-% Asche. Die wässrige Aufschlämmung ist neutral (Norit SX Plus) | 0 |
| Dampfaktiviertes, säuregewaschenes Aktivkohleextrudat 0,8 mm, mit BET-Oberfläche 1100 m²/g, Jodzahl 1000, Methylenblau-Absorption 22 g/100g, Melassezahl 350 [EUR] und 5 Gew-% Asche. Die wässrige Aufschlämmung ist neutral (Norit Rox 0.8) | 3 |
| Chemisch aktivierte, säuregewaschene, stückige Aktivkohle 3 mm mit Bentonit-Zusatz und BET-Oberfläche 1100 m²/g, Methylenblau-Absorption von 25 g/100g, Melassezahl von 350 [EUR] und 15 Gew-% Asche. Die wässrige Aufschlämmung ist sauer (pH 2 bis 3,5) (Norit Bentonorit CA 1) | 3 |

### Beispiel 3:

In zwei Versuchen wurden jeweils 70 g TMP mit einem Gehalt von 99.23 % (GC-Flächen-%) und einer APHA-Farbzahl von 10 aufgeschmolzen und auf eine Temperatur von 100 °C gebracht. Anschließens wurden jeweils 0.35 g eines dampfaktivierten, säuregewaschenen Aktivkohleextrudats 0,8 mm mit einer BET-Oberfläche von 1100 m²/g, einer Jodzahl von 1000, einer Methylenblau-Absorption von 22 g/100g, einer Melassezahl von 350 [EUR] und 5 Gew% Asche, dessen wässrige Aufschlämmung neutral ist, zugesetzt und die Mischung bei dieser Temperatur im ersten Versuch 10 min lang und im zweiten Versuch 40 min lang gerührt. Anschließend wurde jeweils über eine beheizte Drucknutsche heiß abfiltriert und die APHA-Farbzahl bestimmt.

Im Versuch mit 10 min Kontaktzeit wurde eine APHA-Farbzahl des Aktivkohlebehandelten TMP von 0 gemessen.

Im Versuch mit 40 min Kontaktzeit wurde eine APHA-Farbzahl des Aktivkohlebehandelten TMP von 6 gemessen.

### Beispiel 4:

In einem senkrechten Glasrohrreaktor wurden 310.5 g eines dampfaktivierten, säuregewaschenen Aktivkohleextrudats 0,8 mm mit einer BET-Oberftäche von 1100 m²/g, einer Jodzahl von 1000, einer Methylenblau-Absorption von 22 g/100g, einer Melassezahl von 350 [EUR] und 5 Gew% Asche, dessen wässrige Aufschlämmung neutral ist, eingebracht. Durch dieses Aktivkohlebett wurde TMP aus einer auf 120 °C temperierten Vorlage von unten nach oben in geradem Durchgang gepumpt. Die spezifische Belastung betrug 0.76 kg/(l*h). Die Verweilzeit im Aktivkohlebett betrug 55 min. Der Reaktor war auf 120 °C temperiert, die Temperatur am Reaktorausgang betrug ebenfalls 120 °C. Von dem nach Durchgang durch das Aktivkohlebett aufgefangenen TMP wurde in bestimmten Zeitabständen die APHA-Earbzahl bestimmt.

Die APHA-Farbzahl des ersten TMP-Überlaufs ab Versuchsbeginn betrug 0. Nach 3 h geradem Durchlauf wurde eine APHA-Farbzahl von 7 gemessen. Nach 5.25 h geradem Durchlauf wurde eine APHA-Farbzahl von 3 gemessen.

### Beispiel 5: Langzeitversuch

In einem Rohrreaktor (38 mm Innendurchmesser; Länge 800 mm) wurde eine Schicht (500 mm Höhe) von Steatitkugeln (1 mm) eingefüllt. Darüberliegend wurde eine Schicht mit 180 mm Höhe aus 76.5 g eines dampfaktivierten, säuregewaschenen Aktivkohleextrudats 0,8 mm mit einer BET-Oberfläche von 1100 m²/g, einer Jodzahl von 1000, einer Methylenblau-Absorption von 22 g/100g, einer Melassezahl von 350 [EUR] und 5 Gew% Asche, dessen wässrige Aufschlämmung neutral ist, eingefüllt. Durch diese Schüttung wurde TMP aus einer auf 120 °C temperierten Vorlage von unten nach oben in geradem Durchgang gepumpt. Die Farbzahl des TMP in der Vorlage betrug 22. Die spezifische Belassung der Aktivkohle betrug 1.0 kg/(l*h). Die Verweilzeit im Aktivkohlebett betrug 42 min. Der Reaktor war auf 120 °C temperiert, die Temperatur am Reaktorausgang betrug ebenfalls 120 °C. Von dem nach Durchgang durch das Aktivkohlebett aufgefangenen TMP wurde in bestimmten Zeitabständen die APHA-Farbzahl bestimmt, die hier angegebenen Werte sind Mittelwerte aus drei Versuchen.

Die Ergebnisse sind in folgender Tabelle aufgeführt:

| Zeit [h] | TMP nach Reinigung [APHA-Farbzahl] |
|---|---|
| 0 | 2 |
| 1 | 6 |
| 3 | 7 |
| 4 | 6 |
| 28 | 7 |
| 37 | 8 |
| 48 | 10 |
| 52 | 8 |
| 77 | 11 |
| 95 | 7 |
| 101 | 6 |
| 113 | 11 |
| 119 | 7 |
| 137 | 10 |
| 164 | 10 |
| 172 | 8 |
| 196 | 8 |

## Patentansprüche

1. Verfahren zur Farbzahlverbesserung von Trimethylolpropan, **dadurch gekennzeichnet, dass** bei Umgebungsdruck in einem Temperaturbereich von 80-200°C flüssiges Trimethylolpropan, das einen Trimethylolpropan-Gehalt von 90 bis 100 Gew.-% aufweist, mit Aktivkohle in Kontakt gebracht wird und die Aktivkohle anschließend abgetrennt wird.

2. Verfahren nach Anspruch 1, **dadurch gekennzeichnet, dass** als Aktivkohlen solche eingesetzt werden, die aus Holzkohle Braunkohle, Steinkohle, Torf, Holz, Kokosnussschalen oder Olivenkernen hergestellt wurden.

3. Verfahren nach einem der Ansprüche 1 bis 2, **dadurch gekennzeichnet, dass** die Aktivkohle eine BET-Oberflächen von 500 bis 2500 m²/g aufweist.

4. Verfahren nach einem der Ansprüche 1 bis 3, **dadurch gekennzeichnet, dass** das Inkontaktbringen mit Aktivkohle 1 min bis 24 h dauert.

## Claims

1. Method of improving the colour number of trimethylolpropane, **characterized in that** trimethylolpropane which is liquid in the temperature range of 80-200°C at ambient pressure and has a trimethylolpropane content of from 90 to 100% by weight is brought into contact with activated carbon and the activated carbon is subsequently separated off.

2. Method according to Claim 1, **characterized in that** activated carbons which have been produced from wood charcoal, brown coal, hard coal, peat, wood, coconut shells or olive stones are used as activated carbons.

3. Method according to either of Claims 1 and 2, **characterized in that** the activated carbon has a BET surface area of from 500 to 2500 m²/g.

4. Method according to any of Claims 1 to 3, **characterized in that** the contacting with activated carbon takes from 1 min to 24 h.

## Revendications

1. Procédé pour l'amélioration de l'indice de coloration de triméthylolpropane, **caractérisé en ce qu'**on met en contact du triméthylolpropane liquide à pression ambiante dans une plage de température de 80-200°C, qui présente une teneur en triméthylolpropane de 90 à 100% en poids, avec du charbon actif et le charbon actif est ensuite séparé.

2. Procédé selon la revendication 1, **caractérisé en ce qu'**on utilise, comme charbons actifs, ceux qui ont été préparés à partir de charbon de bois, de lignite, d'anthracite, de tourbe, de bois, de coques de noix de coco ou de noyaux d'olives.

3. Procédé selon l'une quelconque des revendications 1 à 2, **caractérisé en ce que** le charbon actif présente une surface BET de 500 à 2500 m²/g.

4. Procédé selon l'une quelconque des revendications 1 à 3, **caractérisé en ce que** la mise en contact avec le charbon actif dure 1 min à 24 h.
